# EUROPÄISCHE PATENTSCHRIFT

(11) **EP 0 711 732 B1**
(45) Veröffentlichungstag und Bekanntmachung des Hinweises auf die Patenterteilung: **04.10.2001**
(21) Anmeldenummer: 95117598.3
(22) Anmeldetag: 08.11.1995
(51) Int. Cl.: C02F 3/28, C02F 11/04, B01D 21/02

(54) **Modul für einen Reaktor zur anaeroben Abwasserreinigung**
Module for a reactor for anaerobic waste water purification
Module pour un réacteur pour la purification anaérobie d'eau usée

(30) Priorität: 08.11.1994 DE 4439802
(43) Veröffentlichungstag der Anmeldung: 15.05.1996
(73) Patentinhaber: USF Deutschland GmbH, 70736 Fellbach (DE)
(72) Erfinder: Rosmanith, Peter, D-78239 Rielasingen (DE)
(74) Vertreter: Grünecker, Kinkeldey, Stockmair & Schwanhäusser Anwaltssozietät

(56) Entgegenhaltungen:
- EP-A- 0 244 029
- EP-A- 0 300 348

## Beschreibung

Ein an Bedeutung zunehmendes Verfahren zur Reinigung von organisch belastetem Abwasser ist das sogenannte UASB-Verfahren (Upflow-Anaerobic-Sludge -Blanket). In dem leicht auftrömenden Abwasser mit einer Geschwindigkeit von unter 1m/h entstehen durch die abbauenden Bakterien als Anreicherung der Biomasse sogenannte Schlammpellets.

Diese sind durch die Umwandlung der organischen Abwasserinhaltsstoffe mit Methanbläschen umgeben. Im Reaktor führt dies daher zu einem Aufströmen der Schlammpellets, bis zur hydraulischen Oberfläche des Reaktors, wo das Methan aufgrund der veränderten Oberflächenspannung abgegeben wird und die Pellets wieder auf den Reaktorboden absinken.

Reaktoren , die nach dem UASB (Upflow-Anaerobic-Sludge-Blanket)-Verfahren arbeiten, werden mit Modulen versehen, die eine Trennung der drei Phasen Bioschlamm (Pellets), Biogas und Wasser durchführen können. Gemäß der Patentschrift EP 0 244 029 B1 wird dabei das entstehende Biogas unterhalb des jeweiligen Abscheiderelementes direkt in einen Gassammelraum geleitet.

Der Übergang des Abscheidersystems muß daher fest mit der Außenwandung verschweisst werden, da ansonsten das Biogas innerhalb des Reaktors entweichen und nicht mehr für eine Verwertung zur Verfügung stehen würde.

Insbesondere durch Transportschäden sowie durch eine unterschiedliche Wärmeausdehnung und Alterung der verschiedenen Materialien kommt es daher leicht zu einer Beschädigung der Schweißnähte und einem Ausfall der Abscheidewirkung.

Weiterhin ist nachteilig, daß überstehende Schlammpartikel durch die direkte Zufuhr zur Gassammelkammer leicht mitgerissen und in den Gassammeiraum getragen werden können. Dieser muß dann aufwendig gereinigt werden.

Ein im Oberbegriff des Anspruchs 1 berücksichtigtes Modul ist aus der DE-A-42 01 864 bekannt. Die Auffanghauben des bekannten Moduls stoßen mit ihren Stirnseiten an eine Begrenzungswand eines Gassammelraumes an. Die Ableitung für das sich unter der Auffanghaube ansammelnde Biogas ist als waagerecht verlaufende Öffnung durch die Wandung des Gassammelraums ausgebildet und liegt über die gesamte Länge des Strömungsweges des Biogases zwischen der Einlaßöffnung im Inneren der Auffanghaube und der Auslaßöffnung im Gassammelraum auf einer Höhe, die zwischen dem First der Auffanghaube und ihren unteren Begrenzungskanten liegt. Die der Auffanghaube zugewandte Seite der Durchtrittsöffnung durch die Wandung des Gassammelraumes ist mit einer Tasche in Form eines nach oben verlaufenden Schachtes abgedeckt, die offensichtlich als Überlauf funktionieren, um Schlamm und Wasser am Übertritt in den Gassammelraum zu hindern. Es hat sich jedoch herausgestellt, daß dies zumindest bei extremen Betriebsbedingungen (geringe Biogasentwicklung) nur unzureichend funktioniert, so daß der Gassammelraum von Zeit zu Zeit aufwendig gereinigt werden muß.

Aus der EP 193 999 B ist eine Anlage zur anaeroben Reinigung von Abwasser bekannt, die einen Tank mit einer Mehrzahl in drei übereinanderliegenden Ebenen angeordneten Auffanghauben für das Biogas aufweist. Das von den Auffanghauben abgefangene, aufsteigende Biogas wird in einer gemeinsamen Haupt-Auslaßleitung gesammelt, die sich oberhalb der obersten Ebene der Auffanghauben und oberhalb der oberen Überlaufschwelle für das gereinigte Abwasser quer über die Mitte des Tanks erstreckt. Die Ableitungen für das gesammelte Biogas aus allen Auffanghauben erstrekken sich über den First der jeweiligen Auffanghaube über ihre gesamte Länge senkrecht nach oben. Dabei haben die Auffanghauben der obersten beiden Ebenen eine direkt Verbindung zur Haupt-Auslaßleitung, während die Ableitung jeder Auffanghaube der untersten Ebene in der jeweils darüberliegenden Auffanghaube der obersten Ebene mündet, die jedoch auch unterhalb der Überlaufschwelle liegt. Auf diese Weise könnte nicht nur Gas sondern auch Schlamm und Wasser aus den Auffanghauben der untersten Ebene in die Auffanghauben der obersten Ebene gelangen. Dies soll bei der bekannten Anlage durch eine zweckmäßige Ausgestaltung des Querschnitts der Ableitung der Auffanghauben der untersten Ebene verhindert werden, was jedoch konstruktiv äußerst aufwendig ist. Darüber hinaus muß durch einen geregelten Gasdruck in der Haupt-Auslaßleitung dafür gesorgt werden, daß sich unter dem First der Auffanghauben immer ein Gaspolster befindet, damit kein Schlamm oder kein Wasser nach oben mitgerissen wird. Auch dieser Druck muß deshalb aufwendig überwacht werden.

Demgegenüber liegt der Erfindung die Aufgabe zugrunde, ein Modul für einen Reaktor zur anaeroben Reinigung von Abwasser bereitzustellen, das konstruktiv einfach aufgebaut ist und eine zuverlässige Trennung des Gases vom Schlamm und vom Wasser gestattet.

Die Aufgabe wird durch die im Anspruch 1 angegebenen Merkmale gelöst.

Durch die erfindungsgemäße Ausgestaltung wird auf konstruktiv einfache Weise eine wirksame Trennung des Gases vom Schlamm bzw. vom Wasser erreicht, so daß eine Verschmutzung des Gassammelraumes und dessen nachfolgende, aufwendige Reinigung vermieden wird.

Bei Anlagen zur Abwasserreinigung, die nach dem UASB (Upflow-Unaerobic-Sludge-Blanket) Verfahren arbeiten, werden alle Teile miteinander verschweißt. Der Gasabzug erfolgt unterhalb der Gassammelhaube, und wird direkt in den Gassammelkanal geleitet. Die erfindungsgemäße Einrichtung erlaubt es dagegen die Gassammelhauben flexibel an den Verankerungen anzubringen. Die Abdichtung erfolgt durch innerhalb der Gassammelhaube spannungsfrei angeklebten oder angeschweißten halbrunden Kunststoffplatten. Die Ableitung des unter der Gassammelhaube aufgefangenen Biogases erfolgt über Rohrleitungen, die mit Biogas gefüllt sind, so ein Gaspolster bilden und ein Eindringen von Schlammpellets in den Gassammelraum wirksam verhindern. Die Zuführung des Biogases in die Gassammelkammer erfolgt oberhalb des jeweiligen Gassammlers.

Die Zeichnungen beinhalten nachfolgend die Einrichtungen:

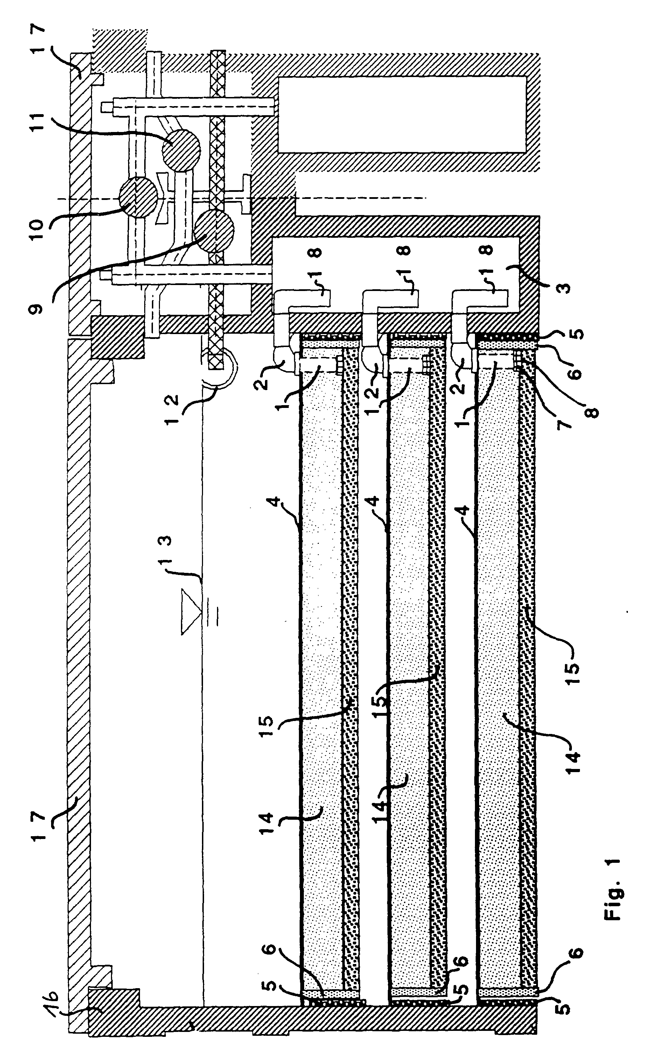

- Fig.1:: Querschnitt durch das Modul

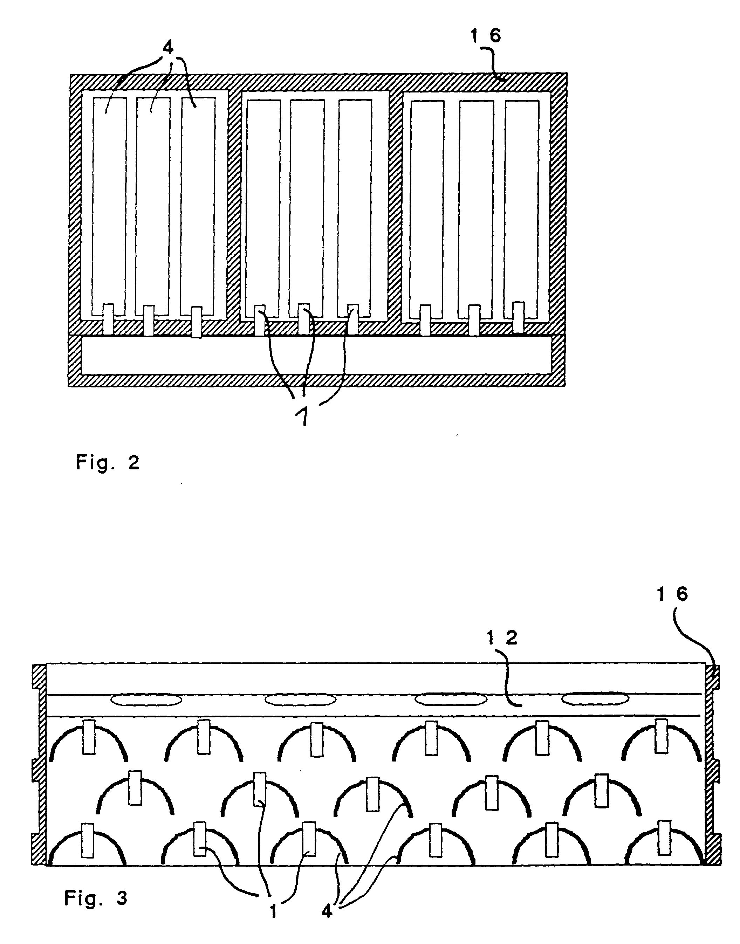

- Fig. 2:: Draufsicht auf das Modul
- Fig.3:: Lägsschnitt durch das Modul

Die erfindungsgemäße Neuerung des vorliegenden Moduls besteht darin, dass das entstandene Gas (14) unterhalb des jeweiligen Abscheiders (4) und oberhalb des Schlammbettes (15) über eine zusätzliche Sedimentationsstrecke (1) und eine gebogene Rohrleitung (2) und gegebenenfalls einer weiteren, nach unten gebogenen Rorleitung (18) dem Gassammelraum (3) oberhalb des jeweiligen Abscheidersystems (4) zugeführt wird und gegebenenfalls die Gassammler (4) frei aufgehängt, lediglich auf Stützprofilen (5) verankert sind. Auf diese Weise entfällt eine anfällige Verschweißung der Sammelhauben mit den Außenwänden des Abscheidermoduls. Zur Abdichtung der Gassammler (4) können diese mit spannungsfrei eingeklebten oder eingeschweißten Halbrunden (6) versehen werden.

Weiterhin können die an der Grenzfläche zum Biogas aufgeschwommenen Schlammpellets durch das in den Rohrleitungen (1,2,18) befindliche Biogas wesentlich besser an einem Übertritt in den Gassammelraum gehindert werden. Eine aufwendige Reinigung des Gassammelraumes (3) entfällt.

Durch eine Einkerbung (7) des unteren Randes der Biogasleitung sowie durch eine Distanzeinrichtung (8) lässt sich die Trenngenauigkeit von Gas und Schlammpellets noch verbessern.

Der Ablauf des behandelten Wassers aus dem Modul erfolgt über eine längsseits angebrachte Ablaufrinne (12).

Die aus gegebenenfalls aus längs halbierten Rohren bestehenden Abscheider (4) sind in ein vorzugsweise aus Kunststoff hergestellten Rahmen (16) eingehängt. Diese Rahmen (16) können mit Stahlprofilen verstärkt sein. Dabei sind die Stahlprofile vollständig kunststoffumhüllt und damit gegen Korrosion geschützt.

Auf der Längsseite des Moduls können die Entsorgungsleitungen für gereinigtes Abwasser (9), Biogas (10) und für Abluft (11) oberhalb des Wasserspiegels geführt werden. Zur Abdeckung des Moduls können ebenfalls Kunststoffplatten (17) eingesetzt werden.

Durch eine abschnittweise Verschließbarkeit der Rohrleitungen kann das einzelne Modul dem Reaktor z.B. zu Wartungszwecken entnommen werden. Der Reaktor muß dazu nicht entleert oder teilentleert werden.

## Patentansprüche

1. Modul für einen Reaktor zur anaeroben Reinigung von Abwasser, mit einer den Wasserstand im Modul festlegenden, oberen Überlaufschwelle (12) für das gereinigte Abwasser, mehreren über den Modulquerschnitt gestaffelt angeordneten Auffanghauben (4) für Biogas,wobei jede Auffanghaube (4) einen First und untere Begrenzungskanten aufweist, einer Ableitung (1, 2, 18) zum Ableiten des unter der Auffanghaube (4) angesammelten Biogases in einen Gassammelraum (3) und einer oberen Abzugsleitung (11) für die von den Auffanghauben (4) nicht erfasste Abluft, **dadurch gekennzeichnet, dass** die Ableitung (1, 2 18) von einer im Inneren der Auffanghaube (4) angeordneten Eintrittsöffnung nach oben über den First der Auffanghaube (4) hinausgeführt ist und über einen wieder nach unten geführten Bereich (18) in den Gassammelraum (3) mündet, so **dass** sich in der Ableitung (1, 2, 18) ein als Schlammsperre wirkendes Gaspolster bildet.

2. Modul nach Anspruch 1, **dadurch gekennzeichnet, dass** die Ableitung (1, 2, 18) als im wesentlichen U-förmige Rohrleitung ausgebildet ist.

3. Modul nach Anspruch 1 oder 2, **dadurch gekennzeichnet, dass** ein Bereich der Ableitung (1, 2, 18) im Inneren der Auffanghaube (4) als Sedimentationsstrecke (1) ausgebildet ist und sich vertikal vom First bis in die Nähe der Begrenzungskanten erstreckt.

4. Modul nach einem der Ansprüche 1 bis 3, **dadurch gekennzeichnet, dass** die Eintrittsöffnung in einer waagrechten Ebene verläuft.

5. Modul nach Anspruch 4, **dadurch gekennzeichnet, dass** die Eintrittsöffnung an ihrem unteren Rand mit einer Einkerbung (7) versehen ist.

6. Modul nach einem der Ansprüche 1 bis 5, **dadurch gekennzeichnet, dass** die Eintrittsöffnung mit einer Distanzeinrichtung (8) versehen ist.

## Claims

1. Module for a reactor for anaerobic waste water purification, with an upper overflow threshold (12) for the purified waste water determining the water level in the module, several collecting hoods (4) for biogas arranged staggered across the module cross-section, each collecting hood (4) having a top ridge and lower limiting edges, a discharge pipe (1, 2, 18) for discharging the biogas collected under the collecting hood (4) into a gas collection chamber (3) and an upper vent pipe (11) for waste air not intercepted by the collecting hoods (4), **characterized in that** the discharge pipe (1, 2, 18) is led out from an inlet opening arranged in the inside of the collecting hood (4) upwards via the top ridge of the collecting hood (4) and discharges into the gas collection chamber (3) via an area (18) leading downwards again, so that a gas cushion acting as a sludge barrier forms in the discharge pipe (1, 2, 18).

2. Module according to claim 1, **characterized in that** the discharge pipe (1, 2, 18) is formed as a substantially U-shaped pipe.

3. Module according to claim 1 or 2, **characterized in that** an area of the discharge pipe (1, 2, 18) in the inside of the collecting hood (4) is formed as a sedimentation section (1) and extends vertically from the top ridge as far as the vicinity of the limiting edges.

4. Module according to one of claims 1 to 3, **characterized in that** the inlet opening runs in a horizontal plane.

5. Module according to claim 4, **characterized in that** the inlet opening is provided at its lower edge with a notch (7).

6. Module according to one of claims 1 to 5, **characterized in that** the inlet opening is provided with a spacer device (8).

## Revendications

1. Module pour un réacteur destiné à la purification anaérobie des eaux usées, comportant un seuil de déversement supérieur (12), déterminant le niveau d'eau dans le module, pour les eaux usées purifiées, plusieurs hottes collectrices (4), disposées de façon étagée au-dessus de la section transversale du module et destinées au biogaz, chaque hotte collectrice (4) présentant un faîte et des arêtes inférieures de limitation, une dérivation (1, 2, 18) permettant d'évacuer le biogaz recueilli sous la hotte collectrice (4) dans une chambre de collecte de gaz (3) et une conduite de décharge (11) pour l'air d'échappement non saisi par les hottes collectrices (4), **caractérisé en ce que** la dérivation (1, 2, 18) est dirigée depuis une ouverture d'entrée disposée à l'intérieur de la hotte collectrice (4) vers le faîte des hottes collectrices (4) et débouche, par une zone (18) qui redirige vers le bas, dans la chambre de collecte de gaz (3), de sorte qu'il se forme dans la dérivation (1, 2, 18) une couche gazeuse agissant en tant que barrière à boues.

2. Module selon la revendication 1, **caractérisé en ce que** la dérivation (1, 2, 18) est conçue en tant que canalisation essentiellement en forme de U.

3. Module selon la revendication 1 ou 2, **caractérisé en ce qu'**une zone de la dérivation (1, 2, 18) à l'intérieur de la hotte collectrice (4) est conçue en tant que tronçon de sédimentation (1) et s'étend verticalement du faîte jusqu'à la proximité des arêtes de limitation.

4. Module selon l'une des revendications 1 à 3, **caractérisé en ce que** l'ouverture d'entrée s'étend dans un plan horizontal.

5. Module selon la revendication 4, **caractérisé en ce que** l'ouverture d'entrée est pourvue au niveau de son bord inférieur d'une entaille (7).

6. Module selon l'une des revendications 1 à 5, **caractérisé en ce que** l'ouverture d'entrée est pourvue d'un dispositif d'écartement (8)
